(19) Europäisches Patentamt
European Patent Office
Office européen des brevets

(11) EP 1 037 906 B1

(12) **EUROPÄISCHE PATENTSCHRIFT**

(45) Veröffentlichungstag und Bekanntmachung des Hinweises auf die Patenterteilung:
**16.02.2005 Patentblatt 2005/07**

(21) Anmeldenummer: **98966267.1**

(22) Anmeldetag: **09.12.1998**

(51) Int Cl.[7]: **C07K 7/64**, A61K 38/04

(86) Internationale Anmeldenummer:
**PCT/EP1998/008003**

(87) Internationale Veröffentlichungsnummer:
**WO 1999/031126 (24.06.1999 Gazette 1999/25)**

(54) **CYCLOPEPTIDDERIVATE MIT INTEGRIN-INHIBITOR-EIGENSCHAFTEN**

CYCLOPEPTIDE DERIVATIVES WITH INTEGRIN INHIBITOR PROPERTIES

DERIVES CYCLOPEPTITIQUES PRESENTANT DES PROPRIETES D'INHIBITION DE L'INTEGRINE

(84) Benannte Vertragsstaaten:
**AT BE CH DE ES FR GB IE IT LI NL SE**

(30) Priorität: **16.12.1997 DE 19755800**

(43) Veröffentlichungstag der Anmeldung:
**27.09.2000 Patentblatt 2000/39**

(73) Patentinhaber: **Biomet Deutschland GmbH
14167 Berlin (DE)**

(72) Erfinder:
- **MEYER, Jörg
D-63329 Egelsbach (DE)**
- **NIES, Berthold
D-64407 Fränkisch-Crumbach (DE)**
- **FINSINGER, Dirk
D-80933 München (DE)**
- **JONCZYK, Alfred
D-64295 Darmstadt (DE)**
- **KESSLER, Horst
D-65842 Schwalbach (DE)**
- **KANTLEHNER, Martin
D-85354 Freising (DE)**

(74) Vertreter: **Gross, Felix, Dr. et al
Patentanwälte
Maikowski & Ninnemann
Postfach 15 09 20
10671 Berlin (DE)**

(56) Entgegenhaltungen:
**EP-A- 0 844 252**
**DE-A- 19 538 741**
**DE-A- 19 725 368**

- **D DELFORGE ET AL.: "Solid-phase synthesis of tailed cyclicc peptides: the use of alpha-allyl-protected aspartic acid leads to aspartimide and tetramethylguanidinium formation " LETTERS IN PEPTIDE SCIENCE, Bd. 3, Mai 1996, Seiten 89-97, XP000206392 Escom, Leiden, the Netherlands**
- **D DELFORGE ET AL.: "Automated solid-phase synthesis of cyclic peptides bearing a side-chain tail designedfor subsequent chemical grafting" ANALYTICAL BIOCHEMISTRY, Bd. 242, November 1996, Seiten 180-186, XP002063394 NEW YORK US in der Anmeldung erwähnt**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 037 906 B1

## Beschreibung

Die Erfindung betrifft Verbindungen der Formel I

$$R\text{-}Q\text{-}X \qquad I$$

worin

R cyclo-(Arg-Gly-Asp-Z), wobei Z in der Seitenkette an Q, oder falls Q fehlt, an X gebunden ist,

Q fehlt, $-[CO-R^1-NH-]_m$, $-[NH-R^1-CO-]_m$, $-[CO-R^1-CO-]_m$, $-(CO-CH_2-O-CH_2CH_2-O-CH_2CH_2-NH-)_n$, $-(NH-CH_2-O-CH_2CH_2-O-CH_2CH_2-CO-)_n$, $-(NH-CH_2CH_2-O-CH_2CH_2-O-CH_2-CO-)_n$ oder $-(CO-CH_2-O-CH_2CH_2-O-CH_2CH_2-NH-)_n-[CO-R^1-NH-]_m$,

X $-CO-CH=CH_2$, $-CO-C(CH_3)=CH_2$, $-NH-CH=CH_2$, $-NH-C(CH_3)=CH_2$ oder $-NH-(CH_2)_p-SR^{10}$,

Z jeweils unabhängig voneinander einen Aminosäurerest oder einen Di- oder Tripeptidrest, wobei die Aminosäuren unabhängig voneinander ausgewählt sind aus einer Gruppe bestehend aus Ala, Asn, Asp, Arg, Cys, Gln, Glu, Gly, His, Homo-Phe, Ile, Leu, Lys, Met, Phe, Phg, Pro, Ser, Thr, Trp, Tyr, Val oder M,
wobei die genannten Aminosäuren auch derivatisiert sein können, und die Aminosäurereste über die α-Amino- und α-Carboxygruppen peptidartig miteinander verknüpft sind. und
wobei M immer enthalten ist,

M $NH(R^8)-CH(R^3)-COOH$,

$R^1$ fehlt oder $R^2$, $R^9$, $R^2-R^9-R^2$, unsubstituiertes oder ein- oder zweifach durch $R^5$ substituiertes Phenylen, wobei die Kettenlänge von $R^5$ jeweils unabhängig voneinander ist,

$R^2$ Alkylen mit 1-10 C-Atomen, wobei 1 oder 2 Methylengruppen durch S, -CH=CH- oder -C≡C- ersetzt sein können,

$R^3$ $-R^5-R^4$, $-R^6-R^4$, $-R^7-R^4$,

$R^4$ OH, $NH_2$, SH oder COOH,

$R^5$ Alkylen mit 1-6 C-Atomen,

$R^6$ Alkylenphenylen mit 7-14 C-Atomen,

$R^7$ Alkylenphenylalkylen mit 8-15 C-Atomen,

$R^8$ H, A oder Alkylenphenyl mit 7-12 C-Atomen,

$R^9$ Cycloalkylen mit 3-7 C-Atomen,

$R^{10}$ H oder eine S-Schutzgruppe,

A Alkyl mit 1-6 C-Atomen,

Hal F, Cl, Br oder I,

m,n jeweils unabhängig voneinander 0, 1, 2 oder 3

und

p 1, 2 oder 3 bedeuten,

wobei, sofern es sich um Reste optisch aktiver Aminosäuren und Aminosäurederivate handelt, sowohl die D- als auch die L-Formen eingeschlossen sind,
sowie deren Salze.

Ähnliche Verbindungen cydischer Peptide sind aus DE 43 10 643 und DE 195 38 741 bekannt.
Die Verbindung cyclo-(Arg-Gly-Asp-Glu(ε-Ahx-Cys-$NH_2$)-D-Val) ist von D.Delforge et al. aus Anal. Biochem. **242,** 180-186 (1996) bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Vor allem wirken sie als Integrin-Inhibitoren, wobei sie insbesondere die Wechselwirkungen der $\alpha_V$-, $\beta_3$- oder $\beta_5$-Integrin-Rezeptoren mit Liganden hemmen, wie z. B. die Bindung von Fibrinogen an den $\beta_3$-Integrinrezeptor. Besondere Wirksamkeit zeigen die Verbindungen im Fall der Integrine $\alpha_V\beta_3$, $\alpha_V\beta_5$, $\alpha_{IIb}\beta_3$ sowie $\alpha_V\beta_1$, $\alpha_V\beta_6$ und $\alpha_V\beta_8$.
Diese Wirkung kann z.B. nach der Methode nachgewiesen werden, die von J.W. Smith et al. in J. Biol. Chem. 265, 12267-12271 (1990) beschrieben wird.
Die Abhängigkeit der Entstehung von Angiogenese von der Wechselwirkung zwischen vaskulären Integrinen und extrazellulären Matrixproteinen ist von P.C. Brooks, R.A. Clark und D.A. Cheresh in Science 264, 569-71 (1994) beschrieben.

Die Möglichkeit der Inhibierung dieser Wechselwirkung und damit zum Einleiten von Apoptose (programmierter Zelltod) angiogener vaskulärer Zellen durch ein cyclisches Peptid ist von P.C. Brooks, A.M. Montgomery, M. Rosenfeld, R.A. Reisfeld, T.-Hu, G. Klier und D.A. Cheresh in Cell 79, 1157-64 (1994) beschrieben.

Verbindungen der Formel I, die die Wechselwirkung von Integrinrezeptoren und Liganden, wie z. B. von Fibrinogen an den Fibrinogenrezeptor (Glycoprotein IIb/IIIa) blockieren, verhindern als GPIIb/IIIa-Antagonisten die Ausbreitung von Tumorzellen durch Metastase. Dies wird durch folgende Beobachtungen belegt:

> Die Verbreitung von Tumorzellen von einem lokalen Tumor in das vaskuläre System erfolgt durch die Bildung von Mikroaggregaten (Mikrothromben) durch Wechselwirkung der Tumorzellen mit Blutplättchen. Die Tumorzellen sind durch den Schutz im Mikroaggregat abgeschirmt und werden von den Zellen des Immunsystems nicht erkannt.
> Die Mikroaggregate können sich an Gefäßwandungen festsetzen, wodurch ein weiteres Eindringen von Tumorzellen in das Gewebe erleichtert wird. Da die Bildung der Mikrothromben durch Fibrinogenbindung an die Fibrinogenrezeptoren auf aktivierten Blutplättchen vermittelt wird, können die GPIIa/IIIb-Antagonisten als wirksame Metastase-Hemmer angesehen werden.

Der (Meth-)acrylatrest dient dazu, die Peptide kovalent an biokompatible Oberflächen von z.B. Implantaten, zu binden, die freie Acrylat- oder Methacrylatreste aufweisen, wie z.B. Polymethylmethacrylatformkörper (Knochenzemente) oder acrylat- bzw. methacrylathaltige Schichten z.B. auf Metalloberflächen.
Entsprechend dient der Thiolrest der Peptidanbindung z.B. an Goldoberflächen.
Gegenstand der Erfindung sind daher insbesondere die Verbindungen der Formel I zur kovalenten Bindung über die funktionelle Gruppe des Restes X an biokompatible Oberflächen.

Im Fall X= -NH-$(CH_2)_p$-$SR^{10}$, ist die funktionelle Gruppe, die an die Oberfläche bindet, der SH-Rest, also wenn $R^{10}$ = H.

Die erfindungsgemäßen Peptide ermöglichen nun die Biofunktionalisierung von Biomaterialien, insbesondere Implantaten für alle denkbaren Organe durch deren Beschichtung, wobei vorwiegend die Adhäsion derjenigen Zellspezies stimuliert wird, die jeweils die Gewebeintegration des entsprechenden Biomaterials vollführen sollen. Mit der Verwendung solcher Beschichtungen ist eine beschleunigte und die verstärkte Integration verschiedener Biomaterialien/Implantate mit verbesserter Langzeitstabilität nach deren Einbringen in den Körper zu erzielen.

Es wird in diesen Zusammenhang auf die am gleichen Tag von der Anmelderin eingereichte zweite Anmeldung verwiesen, in der geeignete Biomaterialien sowie die Beschichtung derselben mit den erfindungsgemäßen Verbindungen, beschrieben sind.

Die erfindungsgemäßen Peptide binden selektiv an Integrine. Nach Immobilisierung an biokompatiblen Oberflächen, z.B. Implantaten, stimulieren sie die Adhäsion von Zellen, die Integrine tragen.
Nach Beschichtung der Verbindungen auf den Oberflächen, können selektiv diejenigen Zell-Spezies zur Bindung stimuliert werden, die auch nach Implantation im natürlichen Gewebe die Implantatintegration vollführen sollen. So handelt es sich z.B. bei Osteoblasten, Osteoclasten und Endothelzellen um $\alpha_V$-tragende Zellspezies.

Gegenstand der Erfindung sind daher die Verbindungen der Formel I als Integrininhibitoren zur selektiven Zellanreicherung an Implantaten.

Die Verbindungen der Formel I können nach Verankerung an einer biokompatiblen Oberfläche als Arzneimittelwirkstoffe in der Human- und Veterinärmedizin eingesetzt werden, insbesondere können sie als Integrininhibitoren

zur Behandlung von durch Implantate verursachten Erkrankungen, Defekten und Entzündungen wie ungenügender und verzögerter Integration von Biomaterialien und Implantaten, von durch Implantate verursachter Thrombose, von Knochen- und Zahndefekten, sowie von osteolytischen Erkrankungen wie Osteoporose, Thrombose, Herzinfarkt, Arteriosklerose, bei der Wundheilung zur Unterstützung der Heilungsprozesse, sowie zur Beschleunigung und Verstärkung des Integrationsprozesses des Implantats bzw. der biokompatiblen Oberfläche in das Gewebe, eingesetzt werden.

Die Verbindungen der Formel I können als antimikrobiell wirkende Substanzen bei Operationen eingesetzt werden, wo Biomaterialien, Implantate, Katheter oder Herzschrittmacher verwendet werden. Dabei wirken sie antiseptisch. Die Wirksamkeit der antimikrobiellen Aktivität kann durch das von P.Valentin-Weigund et al., in Infection and Immunity, 2851-2855 (1988) beschriebene Verfahren nachgewiesen werden.

Gegenstand der Erfindung sind somit die Verbindungen der Formel I als Integrininhibitoren zur Behandlung von durch Implantate verursachten Erkrankungen, Defekten, Entzündungen und von osteolytischen Erkrankungen wie Osteoporose, Thrombose, Herzinfarkt und Arteriosklerose, sowie zur Beschleunigung und Verstärkung des Integrationsprozesses des Implantats bzw. der biokompatiblen Oberfläche in das Gewebe.

Gegenstand der Erfindung ist ferner die Verwendung von Verbindungen der Formel I zur Herstellung eines Arzneimittels zur Behandlung von durch Implantate verursachten Erkrankungen, Defekten, Entzündungen und von osteolytischen Erkrankungen wie Osteoporose, Thrombose, Herzinfarkt und Arteriosklerose, sowie zur Beschleunigung und Verstärkung des Integrationsprozesses des Implantats bzw. der biokompatiblen Oberfläche in das Gewebe.

Entsprechende thiolankertragende Peptide können kovalent an vergoldete Träger, wie z.B. Implantate, Affinitätschromatographien, oder Mikrotiterplatten gebunden werden.

Gegenstand der Erfindung ist auch die Verwendung der neuen Verbindungen der Formel I in der Affinitätschromatographie zum Eluieren von gebundenen Proteinen.

Insbesondere können sie als Integrinliganden zum Eluieren von Integrinen verwendet werden.

Die vor- und nachstehend aufgeführten Abkürzungen von Aminosäureresten stehen für die Reste folgender Aminosäuren:

| | |
|---|---|
| Abu | 4-Aminobuttersäure |
| Aha | 6-Aminohexansäure, 6-Aminocapronsäure |
| Ala | Alanin |
| Asn | Asparagin |
| Asp | Asparaginsäure |
| Arg | Arginin |
| Cys | Cystein |
| Dab | 2,4-Diaminobuttersäure |
| Dap | 2,3-Diaminopropionsäure |
| Gln | Glutamin |
| Glp | Pyroglutaminsäure |
| Glu | Glutaminsäure |
| Gly | Glycin |
| His | Histidin |
| homo-Phe | homo-Phenylalanin |
| Ile | Isoleucin |
| Leu | Leucin |
| Lys | Lysin |
| Met | Methionin |
| Nle | Norleucin |
| Orn | Ornithin |
| Phe | Phenylalanin |
| Phg | Phenylglycin |
| 4-Hal-Phe | 4-Halogen-phenylalanin |
| Pmc | 2,2,5,7,8-Pentamethylchroman-6-sulfonyl |
| Pro | Prolin |
| Ser | Serin |
| Thr | Threonin |

(fortgesetzt)

| | |
|---|---|
| Trp | Tryptophan |
| Tyr | Tyrosin |
| Val | Valin. |

Ferner bedeuten nachstehend:

| | |
|---|---|
| Ac | Acetyl |
| BOC | tert.-Butoxycarbonyl |
| CBZ oder Z | Benzyloxycarbonyl |
| DCCI | Dicyclohexylcarbodiimid |
| DMF | Dimethylformamid |
| EDCI | N-Ethyl-N,N'-(dimethylaminopropyl)-carbodiimid |
| Et | Ethyl |
| FCA | Fluoresceincarbonsäure |
| FITC | Fluoresceinisothiocyanat |
| Fmoc | 9-Fluorenylmethoxycarbonyl |
| FTH | Fluoresceinthioharnstoff |
| HOBt | 1-Hydroxybenzotriazol |
| Me | Methyl |
| MBHA | 4-Methyl-benzhydrylamin |
| Mtr | 4-Methoxy-2,3,6-trimethylphenyl-sulfonyl |
| HONSu | N-Hydroxysuccinimid |
| OBut | tert.-Butylester |
| Oct | Octanoyl |
| OMe | Methylester |
| OEt | Ethylester |
| POA | Phenoxyacetyl |
| Pbf | Pentamethylbenzofuranyl |
| Sal | Salicyloyl |
| Su | Succinyl |
| TFA | Trifluoressigsäure |
| Trt | Trityl (Triphenylmethyl). |

Sofern die vorstehend genannten Aminosäuren in mehreren enantiomeren Formen auftreten können, so sind vor- und nachstehend, z. B. als Bestandteil der Verbindungen der Formel I, alle diese Formen und auch ihre Gemische (z. B. die DL-Formen) eingeschlossen. Ferner können die Aminosäuren, z. B. als Bestandteil von Verbindungen der Formel I, mit entsprechenden an sich bekannten Schutzgruppen versehen sein.
Vor allem Seitenkettenmodifikationen des Arginins, wie sie z.B. bei den nichtpeptidischen $\alpha_V\beta_3$-Antagonisten vorgenommen wurden (z.B. durch R.Keenan et al., Abstr. Pap. 211th ACS National Meeting (New Orleans, USA) 1996, MEDI 236), können auch bei den Cyclopeptiden eingesetzt werden, wie z.B. Benzimidazolderivate anstelle der Guanidingruppe.
In die erfindungsgemäßen Verbindungen sind auch sogenannte Prodrug-Derivate eingeschlossen, d. h. mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.
Gegenstand der Erfindung ist ferner ein Verfahren zur Herstellung von Verbindungen der Formel I nach Anspruch 1 sowie ihrer Salze, dadurch gekennzeichnet, daß man

(a) zur Herstellung von Verbindungen der Formel I,
worin

Q fehlt, $-[CO-R^1-NH-]_m$, $-(CO-CH_2-O-CH_2CH_2-O-CH_2CH_2-NH-)_n$,

oder
$-(CO-CH_2-O-CH_2CH_2-O-CH_2CH_2-NH-)_n-[CO-R^1-NH-]_m$.
X $-CO-CH=CH_2$ oder $-CO-C(CH_3)=CH_2$

bedeuten,
und R die in Anspruch 1 angegebene Bedeutung hat,

i)
eine Verbindung der Formel II

R-H II

worin
R die in Anspruch 1 angegebene Bedeutung hat,
mit einer Verbindung der Formel III

L-Q-X III

worin

Q fehlt, $-[CO-R^1-NH-]_m$, $-(CO-CH_2-O-CH_2CH_2-O-CH_2CH_2-NH-)_n$,
oder
$-(CO-CH_2-O-CH_2CH_2-O-CH_2CH_2-NH-)_n-(CO-R^1-NH-]_m$,
X $-CO-CH=CH_2$ oder $-CO-C(CH_3)=CH_2$

bedeuten,
und

L Cl, Br, I oder eine freie oder reaktionsfähig funktionell abgewandelte OH-Gruppe bedeutet,

umsetzt,
oder

ii)
eine Verbindung der Formel IV

R-Q-H IV

worin

Q fehlt, $-[CO-R^1-NH-]_m$ oder $-(CO-CH_2-O-CH_2CH_2-O-CH_2CH_2-NH-)_n$ oder $-(CO-CH_2-O-CH_2CH_2-O-CH_2CH_2-NH-)_n-[CO-R^1-NH-]_m$,

bedeutet und
R die in Anspruch 1 angegebene Bedeutung hat,
mit einer Verbindung der Formel V

L-X V

worin

X $-CO-CH=CH_2$ oder $-CO-C(CH_3)=CH_2$ bedeutet,

und

L Cl, Br, I oder eine freie oder reaktionsfähig funktionell abgewandelte OH-Gruppe bedeutet,

umsetzt,
oder

(b) zur Herstellung von Verbindungen der Formel I,
worin

Q fehlt, $-[NH-R^1-CO-]_m$, $-[CO-R^1-CO-]_m$, $-(NH-CH_2-O-CH_2CH_2-O-CH_2CH_2-CO-)_n$ oder $-(NH-CH_2CH_2-O-CH_2CH_2-O-CH_2-CO-)_n$,
X $-NH-CH=CH_2$, $-NH-C(CH_3)=CH_2$ oder $-NH-(CH_2)_p-SR^{10}$,
$R^{10}$ eine S-Schutzgruppe

bedeuten,
und R die in Anspruch 1 angegebene Bedeutung hat,

i)
eine Verbindung der Formel VI

R-L VI

worin

L Cl, Br, I oder eine freie oder reaktionsfähig funktionell abgewandelte OH-Gruppe bedeutet,

und R die in Anspruch 1 angegebene Bedeutung hat,
mit einer Verbindung der Formel VII

H-Q-X VII

worin

Q fehlt, $-(NH-R^1-CO-]_m$, $-(NH-CH_2-O-CH_2CH_2-O-CH_2CH_2-CO-)_n$ oder $-(NH-CH_2CH_2-O-CH_2CH_2-O-CH_2-CO-)_n$,
X $-NH-CH=CH_2$, $-NH-C(CH_3)=CH_2$ oder $-NH-(CH_2)_p-SR^{10}$,
$R^{10}$ eine S-Schutzgruppe

bedeuten,
umsetzt,
oder

ii)
eine Verbindung der Formel VIII

R-Q-L VIII

worin

Q fehlt, $-[NH-R^1-CO-]_m$, $-[CO-R^1-CO-]_m$, $-(NH-CH_2-O-CH_2CH_2-O-CH_2CH_2-CO-)_n$ oder $-(NH-CH_2CH_2-O-CH_2CH_2-O-CH_2-CO-)_n$,
L Cl, Br, I oder eine freie oder reaktionsfähig funktionell abgewandelte OH-Gruppe bedeutet,

und R die in Anspruch 1 angegebene Bedeutung hat,
mit einer Verbindung der Formel IX

H-X IX

worin

X $-NH-CH=CH_2$, $-NH-C(CH_3)=CH_2$ oder $-NH-(CH_2)_p-SR^{10}$,
$R^{10}$ eine S-Schutzgruppe

bedeuten,

umsetzt,
oder

c) daß man sie aus einem ihrer funktionellen Derivate durch Behandeln mit einem solvolysierenden oder hydrogenolysierenden Mittel in Freiheit setzt,

und/oder daß man eine basische oder saure Verbindung der Formel I durch Behandeln mit einer Säure oder Base in eines ihrer Salze überführt.

Vor- und nachstehend haben die Reste R, Q, X und L die bei den Formeln I, II, III, IV, V, VI, VII, VIII und IX angegebenen Bedeutungen, sofern nicht ausdrücklich etwas anderes angegeben ist.

In den vorstehenden Formeln steht Alkyl vorzugsweise für Methyl, Ethyl, Propyl, Isopropyl, Butyl, Isobutyl, sek.-Butyl oder tert.-Butyl, ferner auch für Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl.

$R^1$ bedeutet $R^2$, $R^9$, $R^2$-$R^9$-$R^2$, unsubstituiertes oder ein- oder zweifach durch $R^5$ substituiertes Phenylen, wobei die Kettenlänge von $R^5$ jeweils unabhängig voneinander ist, oder $R^1$ fehlt; insbesondere bedeutet $R^1$ Alkylen mit 1-10 C-Atomen.

Alkylen bedeutet bevorzugt Methylen, Ethylen, Propylen, Butylen, Pentylen oder Hexylen, ferner Heptylen, Octylen, Nonylen oder Decylen. Alkylenphenyl ist vorzugsweise Benzyl oder Phenethyl. Alkylenphenylalkylen bedeutet vorzugsweise 4-Methylenbenzyl oder 4-Ethylenbenzyl.

Q bedeutet bevorzugt z.B. den 6-Aminohexansäure (6-Aminocapronsäure)-Rest, den Succinylrest, den $-(CO-CH_2-O-CH_2CH_2-O-CH_2CH_2-NH)-$, den $-(CO-CH_2-O-CH_2CH_2-O-CH_2CH_2-NH-)-CO-(CH_2)_5-NH-$, den $-(CO-(CH_2)_5-NH)_2-$ oder den $-(CO-CH_2-O-CH_2CH_2-O-CH_2CH_2-NH-)_2-CO-(CH_2)_5-NH-$Rest.

M bedeutet vorzugsweise Dap, Ser, Cys, Asp, D-Asp, Dab, Homoserin, Homocystein, Glu, D-Glu, Thr, Orn, Lys, D-Lys, 4-Aminomethyl-Phe oder 4-Aminomethyl-D-Phe.

Die in den Bedeutungen für Z genannten Aminosäuren und Aminosäurereste können auch derivatisiert sein, wobei die N-Methyl-, N-Ethyl-, N-Propyl-, N-Benzyl- oder $C_\alpha$-Methylderivate bevorzugt sind. Weiter bevorzugt sind Derivate von Asp und Glu, insbesondere die Methyl-, Ethyl, Propyl, Butyl, tert.-Butyl, Neopentyl- oder Benzylester der Seitenkettencarboxy-gruppen, ferner auch Derivate von Arg, das an der $-NH-C(=NH)-NH_2$-Gruppe mit einem Acetyl-, Benzoyl-, Methoxycarbonyl- oder Ethoxycarbonylrest substituiert sein kann.

Z bedeutet vorzugsweise M, weiter bevorzugt Homo-Phe-M, Phenylglycin, D-Phe-M, D-Trp-M, D-Tyr-M, D-Phe-Lys, D-Phe-D-Lys, D-Trp-Lys, D-Trp-D-Lys, D-Tyr-Lys, D-Tyr-D-Lys, D-Phe-Orn, D-Phe-Dab, D-Phe-Dap, D-Phe-D-Orn, D-Phe-D-Dab, D-Phe-D-Dap, D-Phe-4-Aminomethyl-Phe, D-Phe-4-Aminomethyl-D-Phe, D-Trp-4-Aminomethyl-Phe, D-Trp-4-Aminomethyl-D-Phe, D-Tyr-4-Aminomethyl-Phe, D-Tyr-4-Aminomethyl-D-Phe, D-Phe-Asp, D-Phe-D-Asp, D-Trp-Asp, D-Trp-D-Asp, D-Tyr-Asp, D-Tyr-D-Asp, D-Phe-Cys, D-Phe-D-Cys, D-Trp-Cys, D-Trp-D-Cys, D-Tyr-Cys, D-Tyr-D-Cys, Phe-D-Lys, Trp-D-Lys, Tyr-D-Lys, Phe-Orn, Phe-Dab, Phe-Dap, Trp-Orn, Trp-Dab, Trp-Dap, Tyr-Orn, Tyr-Dab, Tyr-Dap, Phe-4-Aminomethyl-D-Phe, Trp-4-Aminomethyl-D-Phe, Tyr-4-Aminomethyl-D-Phe, Phe-D-Asp, Trp-D-Asp, Tyr-D-Asp, Phe-D-Cys, Trp-D-Cys, Tyr-D-Cys, Phg-M, D-Phe-Lys-Gly, D-Phe-M-Gly, D-Trp-Lys-Gly, D-Trp-M-Gly, D-Tyr-Lys-Gly, D-Tyr-M-Gly, D-Phe-Val-Lys, D-Phe-Gly-Lys, D-Phe-Ala-Lys, D-Phe-Ile-Lys, D-Phe-Leu-Lys, D-Trp-Val-Lys, D-Trp-Gly-Lys, D-Trp-Ala-Lys, D-Trp-Ile-Lys, D-Trp-Leu-Lys, D-Tyr-Val-Lys, D-Tyr-Gly-Lys, D-Tyr-Ala-Lys, D-Tyr-Ile-Lys, D-Tyr-Leu-Lys.

Der Rest $-R^6-R^4$ bedeutet bevorzugt 2-, 3- oder 4-Hydroxybenzyl, 2-, 3-oder 4-Aminobenzyl, 2-, 3- oder 4-Mercaptobenzyl, 2-, 3- oder 4- Carboxybenzyl, ferner bevorzugt 2-, 3- oder 4-Hydroxyphenethyl, 2-,3- oder 4-Aminophenethyl, 2-, 3- oder 4- Mercaptophenethyl, 2-, 3- oder 4-Carboxyphenethyl.

Cycloalkylen bedeutet bevorzugt Cyclopropylen, 1,2- oder 1,3-Cyclobutylen, 1,2- oder 1,3-Cyclopentylen, 1,2- , 1,3- oder 1,4-Cyclohexylen, ferner 1,2- , 1,3- oder 1,4-Cycloheptylen.

$R^{10}$ bedeutet H oder eine S-Schutzgruppe, wie z.B. Trityl.

Aminoschutzgruppe bedeutet vorzugsweise Acetyl, Propionyl, Butyryl, Phenylacetyl, Benzoyl, Toluyl, POA,

Methoxycarbonyl, Ethoxycarbonyl, 2,2,2-Trichlorethoxycarbonyl, BOC, 2-Iodethoxycarbonyl, CBZ ("Carbobenzoxy"), 4-Methoxybenzyloxycarbonyl, FMOC, Mtr, Benzyl, Pbf oder Pmc.

Hal bedeutet vorzugsweise F, Cl oder Br, aber auch I.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis If ausgedrückt werden, die der Formel I entsprechen und worin die nicht näher bezeichneten Reste die bei der Formel I angegebene Bedeutung haben, worin jedoch

| | | | |
|---|---|---|---|
| in | a) | Z | D-Phe-M, Phe-M, D-Trp-M, Trp-M, D-Tyr-M, Tyr-M, D-Phg-M, Phg-M, D-Homo-Phe-M, Homo-Phe-M oder D-Phe-Lys-Gly bedeutet; |
| in | b) | Z<br>X | D-Phe-M, Phe-M, D-Trp-M, Trp-M, D-Tyr-M, Tyr-M, D-Phg-M, Phg-M, D-Homo-Phe-M, Homo-Phe-M oder D-Phe-Lys-Gly,<br>$-CO-CH=CH_2$, $-NH-CH=CH_2$, oder $-NH-(CH_2)_p-SH$, bedeuten; |
| in | c) | Z<br>X | D-Phe-M, Phe-M, D-Trp-M, Trp-M, D-Tyr-M, Tyr-M, D-Phg-M, Phg-M, D-Homo-Phe-M, Homo-Phe-M oder D-Phe-Lys-Gly,<br>$-CO-CH=CH_2$ oder $-NH-(CH_2)_p-SH$, bedeuten; |
| in | d) | Z<br>X<br>$R^1$ | D-Phe-M, Phe-M, D-Trp-M, Trp-M, D-Tyr-M, Tyr-M, D-Phg-M, Phg-M, D-Homo-Phe-M, Homo-Phe-M oder D-Phe-Lys-Gly,<br>$-CO-CH=CH_2$, $-NH-CH=CH_2$ oder $-NH-(CH_2)_p-SH$,<br>$R^2$,<br>bedeuten; |
| in | e) | R<br>X | Cyclo-(Arg-Gly-Asp-D-Phe-M) oder D-Phe-Lys-Gly,<br>$-CO-CH=CH_2$, $-NH-CH=CH_2$ oder $-NH-(CH_2)_p-SH$, bedeuten; |
| in | f) | R<br>Q<br><br><br>X<br>$R^1$ | Cyclo-(Arg-Gly-Asp-D-Phe-M) oder D-Phe-Lys-Gly,<br>$-CO-(CH_2)_5-NH$, $-CO-(CH_2)_2-CO-$, $-CO-CH_2-O-CH_2CH_2-O-CH_2CH_2-NH-$ oder $-(CO-CH_2-O-CH_2CH_2-O-CH_2CH_2-NH-)_2-CO-(CH_2)_5-NH$, $-(CO-CH_2-O-CH_2CH_2-O-CH_2CH_2-NH-)_1-CO-(CH_2)_5-NH-$, $-(CO-(CH_2)_5-NH)_2-$,<br>$-CO-CH=CH_2$, $-NH-CH=CH_2$ oder $-NH-(CH_2)_p-SH$,<br>$R^2$,<br>bedeuten. |

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart;) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Verbindungen der Formel I können vorzugsweise erhalten werden, indem man Verbindungen der Formel II mit Verbindungen der Formel III umsetzt.

Die Verbindungen der Formel II und III sind in der Regel bekannt. Sind sie nicht bekannt, so können sie nach an sich bekannten Methoden hergestellt werden.

In den Verbindungen der Formel III bedeutet der Rest L vorzugsweise eine voraktivierte Carbonsäure, vorzugsweise ein Carbonsäurehalogenid, symmetrisches oder gemischtes Anhydrid oder einen Aktivester. Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben. Aktivierte Ester werden zweckmäßig in situ gebildet, z. B. durch Zusatz von HOBt oder N-Hydroxysuccinimid.

L bedeutet vorzugsweise H, F, Cl, Br oder -ON-Succinimid.

Die Umsetzung erfolgt in der Regel in einem inerten Lösungsmittel, in Gegenwart eines säurebindenden Mittels vorzugsweise einer organischen Base wie DIPEA, Triethylamin, Dimethylanilin, Pyridin oder Chinolin oder eines Überschusses der Carboxykomponente der Formel III.

Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, N-Methylpyrrolidon, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat, Wasser oder Gemische der genannten Lösungsmittel.

Verbindungen der Formel I können weiterhin erhalten werden, indem man Verbindungen der Formel IV mit Verbindungen der Formel V umsetzt.

Die Ausgangsverbindungen der Formel IV und V sind in der Regel bekannt. Sind sie nicht bekannt, so können sie nach an sich bekannten Methoden hergestellt werden.

In den Verbindungen der Formel V bedeutet der Rest L vorzugsweise eine voraktivierte Carbonsäure, vorzugsweise ein Carbonsäurehalogenid, symmetrisches oder gemischtes Anhydrid oder einen Aktivester. Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.

L bedeutet vorzugsweise F, Cl, Br oder -ON-Succinimid.

Die Umsetzung der Verbindungen der Formel IV mit Verbindungen der Formel V erfolgt unter den gleichen Bedingungen, betreffend die Reaktionszeit, Temperatur und Lösungsmittel, wie dies für die Umsetzung der Verbindungen der Formel II mit Verbindungen der Formel III beschrieben ist.

Verbindungen der Formel I können weiterhin erhalten werden, indem man Verbindungen der Formel VI mit Verbindungen der Formel VII umsetzt. Die Ausgangsverbindungen der Formel VI und VII sind in der Regel bekannt. Sind sie nicht bekannt, so können sie nach an sich bekannten Methoden hergestellt werden.

In den Verbindungen der Formel VI bedeutet der Rest L vorzugsweise eine voraktivierte Carbonsäure, vorzugsweise ein Carbonsäurehalogenid, symmetrisches oder gemischtes Anhydrid oder einen Aktivester. Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.

L bedeutet vorzugsweise F, Cl, Br oder -ON-Succinimid.

Die Umsetzung der Verbindungen der Formel VI mit Verbindungen der Formel VII erfolgt unter den gleichen Bedingungen, betreffend die Reaktionszeit, Temperatur und Lösungsmittel, wie dies für die Umsetzung der Verbindungen der Formel II mit Verbindungen der Formel III beschrieben ist.

Verbindungen der Formel I können weiterhin erhalten werden, indem man Verbindungen der Formel VIII mit Verbindungen der Formel IX umsetzt. Die Ausgangsverbindungen der Formel VIII und IX sind in der Regel bekannt. Sind sie nicht bekannt, so können sie nach an sich bekannten Methoden hergestellt werden.

In den Verbindungen der Formel VIII bedeutet der Rest L vorzugsweise eine voraktivierte Carbonsäure, vorzugsweise ein Carbonsäurehalogenid, symmetrisches oder gemischtes Anhydrid oder einen Aktivester. Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.

L bedeutet vorzugsweise F, Cl, Br oder -ON-Succinimid.

Die Umsetzung der Verbindungen der Formel VIII mit Verbindungen der Formel IX erfolgt unter den gleichen Bedingungen, betreffend die Reaktionszeit, Temperatur und Lösungsmittel, wie dies für die Umsetzung der Verbindungen der Formel II mit Verbindungen der Formel III beschrieben ist.

Cyclische Verbindungen der Formel II können durch Cyclisierung der linearen Verbindungen hergestellt werden, wie z. B. in DE 43 10 643, in Houben-Weyl, l.c., Band 15/II, Seiten 1 bis 806 (1974) oder von S. Zimmer E. Hoffmann, G. Jung und H. Kessler, Liebigs Ann. Chem. **1993,** 497-501, beschrieben.

Die linearen Peptide können z.B. nach R.B. Merrifield, Angew. Chemie **1985,** 97, 801-812, synthetisiert werden.

Offenkettige lineare Verbindungen, wie z.B. Verbindungen der Formel III können im übrigen nach üblichen

Methoden der Aminosäure- und Peptidsynthese hergestellt werden, z. B. auch nach der Festphasensynthese nach Merrifield (s. auch z.B. B. F. Gysin und R. B. Merrifield, J. Am. Chem. Soc. 94, 3102 ff. (1972)).

Die Verbindungen der Formeln I können ferner erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z. B. solche, die der Formel I entsprechen, aber anstelle einer $NH_2$-Gruppe eine NHR'-Gruppe (worin R' eine Aminoschutzgruppe bedeutet, z. B. BOC oder CBZ) enthalten.

Ferner sind Ausgangsstoffe bevorzugt, die anstelle des H-Atoms einer Hydroxygruppe eine Hydroxyschutzgruppe tragen, z. B. solche, die der Formel I entsprechen, aber anstelle einer Hydroxyphenylgruppe eine R"O-phenylgruppe enthalten (worin R" eine Hydroxyschutzgruppe bedeutet).

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-Iodethoxycarbonyl; Aralkyloxycarbonyl wie CBZ ("Carbobenzoxy"), 4-Methoxybenzyloxycarbonyl, FMOC; Arylsulfonyl wie Mtr, Pbf oder Pmc. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl, tert.-Butyl und Acetyl, wobei Benzyl und tert.-Butyl besonders bevorzugt sind.

Die COOH-Gruppen in Asparaginsäure und Glutaminsäure werden bevorzugt in Form ihrer tert.-Butylester geschützt (z. B. Asp(OBut)).

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut, Pbf, Pmc und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Die Tritylgruppe wird zum Schutz der Aminosäuren Histidin, Asparagin, Glutamin und Cystein eingesetzt. Die Abspaltung erfolgt, je nach gewünschtem Endprodukt, mit TFA / 10% Thiophenol, wobei die Tritylgruppe von allen genannten Aminosäuren abgespalten wird, bei Einsatz von TFA / Anisol oder TFA / Thioanisol wird nur die Tritylgruppe von His, Asn und Gln abgespalten, wogegen sie an der Cys-Seitenkette verbleibt. Die Pbf (Pentamethylbenzofuranyl)-gruppe wird zum Schutz von Arg eingesetzt. Die Abspaltung erfolgt z.B. mit TFA in Dichlormethan.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits kann eine Säure der Formel I durch Umsetzung mit einer Base in eines ihrer physiologisch unbedenklichen Metall- oder Ammoniumsalze übergeführt werden. Als Salze kommen dabei insbesondere die Natrium-, Kalium-, Magnesium-, Calcium- und Ammoniumsalze in Betracht, ferner substituierte Ammoniumsalze, z. B. die Dimethyl-, Diethyloder Diisopropyl-ammoniumsalze, Monoethanol-, Diethanol- oder Diisopropylammoniumsalze, Cyclohexyl-, Dicyclohexylammoniumsalze, Dibenzylethylendiammoniumsalze, weiterhin z. B. Salze mit Arginin oder Lysin.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel:

Ethylacetat/Methanol 9:1.
RZ = Retentionszeit (Minuten) bei HPLC in den folgenden Systemen:

[A]

| | |
|---|---|
| Säule | YMC ODS A RP $5C_{18}$, 250 x 4,6 mm |
| Eluent A | 0,1 % TFA in Wasser |
| Eluent B | 0,1 % TFA in Acetonitril |
| Fluß | 1 ml/min |
| Gradient | 0 - 50 % B / 30 min. |

[B]

wie [A];
Gradient: 5 - 50 % B / 30 min.

[C]

wie [A];
Gradient: 10 - 50 % B / 30 min.

| | |
|---|---|
| Massenspektrometrie (MS) | EI (Elektronenstoß-Ionisation) $M^+$<br>FAB (Fast Atom Bombardment) $(M+H)^+$<br>ESI (Elektrospray-Ionisation) $(M+H)^+$ |

DMPP-Harz steht für 4-(2',4'-Dimethoxyphenyl-hydroxymethyl)phenoxy-Harz, welches z.B. die Synthese von seitenkettengeschützten Peptiden erlaubt, TCP-Harz bedeutet Tritylchlorid-Polystyrol-Harz.

Beispiel 1

a) Zu einer Lösung aus 0,1 mmol Cyclo-(Arg(Pbf)-Gly-Asp(OBut)-D-Phe-Lys) ("A") [erhältlich durch Cyclisierung von H-Asp(OBut)-D-Phe-Lys(Z)-Arg(Pbf)-Gly-OH zu Cyclo-(Arg(Pbf)-Gly-Asp(OBut)-D-Phe-Lys(Z)) und selektiver Abspaltung der Z-Gruppe durch Hydrogenolyse] in 5 ml DMF gibt man 0,2 mmol Bernsteinsäureanhydrid.
Man rührt 5 Stunden bei Raumtemperatur und erhält nach üblicher Aufarbeitung Cyclo-(Arg(Pbf)-Gly-Asp(OBut) -D-Phe-Lys($N^\varepsilon$-Su)) ("B").

b) 1 Äquivalent Cysteaminhydrochlorid und 1Äquivalent Triphenylmethanol werden bei 60° in Eisessig gelöst und unter Rühren mit 1,1 Äquivalent $BF_3$-Etherat versetzt. Man rührt eine Stunde nach, arbeitet wie üblich auf und erhält Trt-Cysteaminhydrochlorid ("C").

Durch Umsetzung von "B" mit "C" in Dichlormethan unter Zusatz von EDCI-hydrochlorid erhält man nach üblicher Aufarbeitung Cyclo-(Arg(Pbf)-Gly-Asp(OBut)-D-Phe-Lys($N^\varepsilon$-CO-$CH_2CH_2$CO-NH-$CH_2CH_2$-S-Trt)).
Nach Abspaltung der Schutzgruppen erhält man
Cyclo-(Arg-Gly-Asp-D-Phe-Lys($N^\varepsilon$-CO-$CH_2CH_2$CO-NH-$CH_2CH_2$-SH)); RZ [B] 18.3; FAB 763.

Analog erhält man durch Umsetzung von "C" mit Cyclo-(Arg(Pbf)-Gly-Asp(OBut)-D-Phe-Lys($N^\varepsilon$-Su)-Gly) und Abspaltung der Schutzgruppen die Verbindung
Cyclo-(Arg-Gly-Asp-D-Phe-Lys($N^\varepsilon$-CO-$CH_2CH_2$CO-NH-$CH_2CH_2$-SH)-Gly).

Beispiel 2

a) Zu einer Suspension von 10 mmol 6-Aminohexansäure und 1,8 Äquivalenten Calciumhydroxid in Wasser gibt man bei 0° 1,2 Äquivalente Acrylsäurechlorid. Man filtriert und erhält nach üblicher Aufarbeitung 6-Acrylamidohexansäure ("D").

b) Eine Lösung von 10 mmol "D" und 1 Äquivalent N-Hydroxysuccinimid in 50 ml Dichlormethan wird bei 0° mit 1,2 Äquivalenten EDCI-hydrochlorid versetzt und 1 Stunde gerührt. Man gibt 10 µl Eisessig hinzu, arbeitet wie üblich auf und erhält 6-Acrylamido-hexansäure HOBTester ("E").

c) Durch Umsetzung von "A" mit "E" in DMF erhält man nach üblicher Aufarbeitung Cyclo-(Arg(Pbf)-Gly-Asp(OBut) -D-Phe-Lys($N^\varepsilon$-CO-$(CH_2)_5$-NH-CO-CH=$CH_2$)).
Nach Abspaltung der Schutzgruppen erhält man Cyclo-(Arg-Gly-Asp-D-Phe-Lys($N^\varepsilon$-CO-$(CH_2)_5$-NH-CO-CH=$CH_2$)); RZ [A] 22.8; ESI 771.

Analog erhält man die Verbindung Cyclo-(Arg-Gly-Asp-D-Phe-Lys($N^\varepsilon$-CO-$(CH_2)_5$-NH-CO-CH=$CH_2$)-Gly).

Beispiel 3

a) Zu einer Lösung von 10 mmol 6-Aminohexansäure in wässrigem Natriumphosphatpuffer (pH 8) gibt man bei 0° 2 mmol "E", gelöst in Ethanol/Chloroform. Man filtriert und erhält nach üblicher Aufarbeitung 6-(6-Acrylamido-hexanoylamido)-hexansäure ("F")

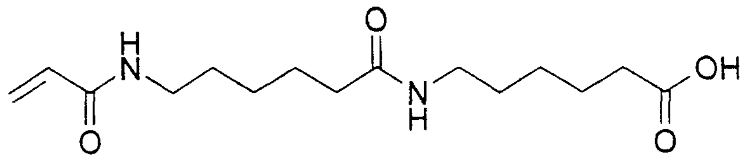

b) Durch Umsetzung von "A" mit "F" in DMF unter Zusatz von EDCI-hydrochlorid erhält man nach üblicher Aufarbeitung Cyclo-(Arg(Pbf)-Gly-Asp(OBut)-D-Phe-Lys($N^{\varepsilon}$-CO-$(CH_2)_5$-NH-CO-$(CH_2)_5$-NH-CO-CH=$CH_2$)). Nach Abspaltung der Schutzgruppen erhält man Cyclo-(Arg-Gly-Asp-D-Phe-Lys($N^{\varepsilon}$- CO-$(CH_2)_5$-NH-CO-$(CH_2)_5$-NH-CO-CH=$CH_2$)); RZ [C] 23.5; ESI 884.

Analog erhält man die Verbindung Cyclo-(Arg-Gly-Asp-D-Phe-Lys($N^{\varepsilon}$- CO-$(CH_2)_5$-NH-CO-$(CH_2)_5$-NH-CO-CH=$CH_2$)-Gly).

Beispiel 4

a) Durch Kupplung von 6-Acrylamidohexansäure an [2-(2-Aminoethoxy)-ethoxy]-essigsäure-TCP-Harz unter Zusatz von HOBt und TBTU in NMP erhält man {2-[2-(6-Acrylamido-hexanoylamido)-ethoxy]-ethoxy}essigsäure-DMPP-Harz.

Man wäscht das Harz mit Dichlormethan/Essigsäure/Trifluorethanol (6:3: 1) und erhält {2-[2-(6-Acrylamido-hexanoylamido)-ethoxy]-ethoxy}-essigsäure ("G").

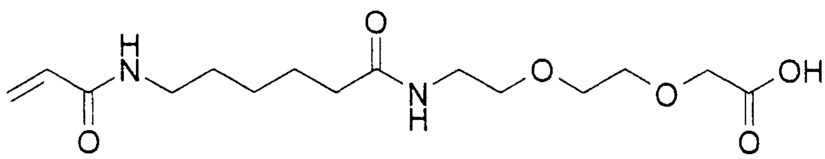

b) Durch Umsetzung von "G" mit "A" in DMF unter Zusatz von EDCIxHCl erhält man die Verbindung Cyclo-(Arg (Pbf)-Gly-Asp(OBut)-D-Phe-Lys($N^{\varepsilon}$-CO-$CH_2$-O-$CH_2CH_2$-O-$CH_2CH_2$-NH-CO-$(CH_2)_5$-NH-CO-CH=$CH_2$)).

Nach Abspaltung der Schutzgruppen erhält man Cyclo-(Arg-Gly-Asp-D-Phe-Lys($N^{\varepsilon}$-CO-$CH_2$-O-$CH_2CH_2$-O-$CH_2CH_2$-NH-CO-$(CH_2)_5$-NH-CO-CH=$CH_2$)); RZ [C] 22.5; ESI 916.

Analog erhält man die Verbindung Cyclo-(Arg-Gly-Asp-D-Phe-Lys($N^{\varepsilon}$-CO-$CH_2$-O-$CH_2CH_2$-O-$CH_2CH_2$-NH-CO-$(CH_2)_5$-NH-CO-CH=$CH_2$)-Gly).

Analog erhält man durch Umsetzung von {2-[2-(2-{2-[2-(6-Acrylamido-hexanoylamido)-ethoxy]-ethoxy}-acetylamido)-ethoxy]-ethoxy}-essigsäure

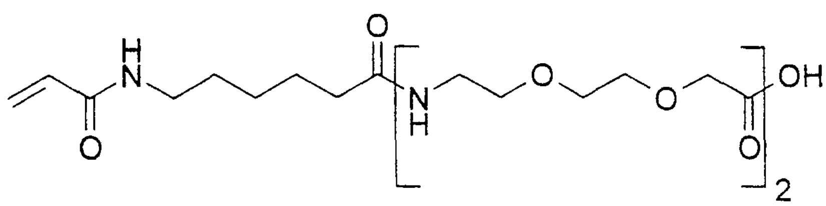

mit "A" die Verbindung Cyclo-(Arg(Pbf)-Gly-Asp(OBut)-D-Phe-Lys($N^{\varepsilon}$-(CO-$CH_2$-O-$CH_2CH_2$-O-$CH_2CH_2$-NH)$_2$-CO-$(CH_2)_5$-NH-CO-CH=$CH_2$)).

Nach Abspaltung der Schutzgruppen erhält man Cyclo-(Arg-Gly-Asp-D-Phe-Lys($N^{\varepsilon}$-(CO-$CH_2$-O-$CH_2CH_2$-O-$CH_2CH_2$-NH)$_2$-CO-$(CH_2)_5$-NH-CO-CH=$CH_2$)); RZ [C] 22.75; ESI 1061.

Analog erhält man Cyclo-(Arg-Gly-Asp-D-Phe-Lys($N^{\varepsilon}$-(CO-$CH_2$-O-$CH_2CH_2$-O-$CH_2CH_2$-NH)$_2$-CO-$(CH_2)_5$-NH-CO-CH=$CH_2$)-Gly).

Beispiel 5

Analog Beispiel 1 erhält man durch Umsetzung der nachstehenden ("G")-Cyclopeptide

Cyclo-(Arg(Pbf)-Gly-Asp(OBut)-D-Phe-Val-Lys)
Cyclo-(Arg(Pbf)-Gly-Asp(OBut)-D-Trp-Lys)
Cyclo-(Arg(Pbf)-Gly-Asp(OBut)-D-Tyr-Lys)
Cyclo-(Arg(Pbf)-Gly-Asp(OBut)-D-Phe-D-Lys)
Cyclo-(Arg(Pbf)-Gly-Asp(OBut)-D-Phe-Cys)
Cyclo-(Arg(Pbf)-Gly-Asp(OBut)-D-Phe-Dab)
Cydo-(Arg(Pbf)-Gly-Asp(OBut)-D-Trp-D-Cys)
Cyclo-(Arg(Pbf)-Gly-Asp(OBut)-D-Tyr-D-Cys)
Cyclo-(Arg(Pbf)-Gly-Asp(OBut)-Phe-D-Lys)
Cyclo-(Arg(Pbf)-Gly-Asp(OBut)-Trp-D-Lys)
Cyclo-(Arg(Pbf)-Gly-Asp(OBut)-Tyr-D-Lys)
Cyclo-(Arg(Pbf)-Gly-Asp(OBut)-Phe-D-Cys)
Cyclo-(Arg(Pbf)-Gly-Asp(OBut)-Phe-Dab)
Cyclo-(Arg(Pbf)-Gly-Asp(OBut)-Trp-D-Cys)
Cyclo-(Arg(Pbf)-Gly-Asp(OBut)-Tyr-D-Cys)
Cyclo-(Arg(Pbf)-Gly-Asp(OBut)-D-Trp-Orn)
Cyclo-(Arg(Pbf)-Gly-Asp(OBut)-D-Tyr-Orn)
Cyclo-(Arg(Pbf)-Gly-Asp(OBut)-D-Phe-Orn)
Cyclo-(Arg(Pbf)-Gly-Asp(OBut)-D-Trp-D-Orn)
Cyclo-(Arg(Pbf)-Gly-Asp(OBut)-D-Tyr-D-Orn)
Cyclo-(Arg(Pbf)-Gly-Asp(OBut)-D-Phe-D-Orn)
Cyclo-(Arg(Pbf)-Gly-Asp(OBut)-D-Trp-Dab)
Cyclo-(Arg(Pbf)-Gly-Asp(OBut)-D-Tyr-Dab)
Cyclo-(Arg(Pbf)-Gly-Asp(OBut)-D-Trp-Dap)
Cyclo-(Arg(Pbf)-Gly-Asp(OBut)-D-Tyr-Dap)
Cyclo-(Arg(Pbf)-Gly-Asp(OBut)-D-Phe-Dap)
Cyclo-(Arg(Pbf)-Gly-Asp(OBut)-D-Trp-D-Dap)
Cyclo-(Arg(Pbf)-Gly-Asp(OBut)-D-Tyr-D-Dap)
Cyclo-(Arg(Pbf)-Gly-Asp(OBut)-D-Phe-D-Dap)

mit
a) Bernsteinsäureanhydrid, b) "C" und c) Abspaltung der Schutzgruppen
die nachfolgenden Verbindungen

Cyclo-(Arg-Gly-Asp-D-Phe-Val-Lys($N^{\varepsilon}$-CO-$CH_2CH_2$CO-NH-$CH_2CH_2$-SH))
Cyclo-(Arg-Gly-Asp-D-Trp-Lys($N^{\varepsilon}$-CO-$CH_2CH_2$CO-NH-$CH_2CH_2$-SH))
Cyclo-(Arg-Gly-Asp-D-Tyr-Lys($N^{\varepsilon}$-CO-$CH_2CH_2$CO-NH-$CH_2CH_2$-SH))
Cyclo-(Arg-Gly-Asp-D-Phe-D-Lys($N^{\varepsilon}$-CO-$CH_2CH_2$CO-NH-$CH_2CH_2$-SH))
Cyclo-(Arg-Gly-Asp-D-Phe-Cys(S-CO-$CH_2CH_2$CO-NH-$CH_2CH_2$-SH))
Cyclo-(Arg-Gly-Asp-D-Phe-Dab($N^{\gamma}$-CO-$CH_2CH_2$CO-NH-$CH_2CH_2$-SH))
Cyclo-(Arg-Gly-Asp-D-Trp-D-Cys(S-CO-$CH_2CH_2$CO-NH-$CH_2CH_2$-SH))
Cyclo-(Arg-Gly-Asp-D-Tyr-D-Cys(S-CO-$CH_2CH_2$CO-NH-$CH_2CH_2$-SH))
Cyclo-(Arg-Gly-Asp-Phe-D-Lys($N^{\varepsilon}$-CO-$CH_2CH_2$CO-NH-$CH_2CH_2$-SH))
Cyclo-(Arg-Gly-Asp-Trp-D-Lys($N^{\varepsilon}$-CO-$CH_2CH_2$CO-NH-$CH_2CH_2$-SH))
Cyclo-(Arg-Gly-Asp-Tyr-D-Lys($N^{\varepsilon}$-CO-$CH_2CH_2$CO-NH-$CH_2CH_2$-SH))
Cyclo-(Arg-Gly-Asp-Phe-D-Cys(S-CO-$CH_2CH_2$CO-NH-$CH_2CH_2$-SH))
Cyclo-(Arg-Gly-Asp-Phe-Dab($N^{\gamma}$-CO-$CH_2CH_2$CO-NH-$CH_2CH_2$-SH))
Cyclo-(Arg-Gly-Asp-Trp-D-Cys(S-CO-$CH_2CH_2$CO-NH-$CH_2CH_2$-SH))
Cyclo-(Arg-Gly-Asp-Tyr-D-Cys(S-CO-$CH_2CH_2$CO-NH-$CH_2CH_2$-SH))
Cyclo-(Arg-Gly-Asp-D-Trp-Orn($N^{\delta}$-CO-$CH_2CH_2$CO-NH-$CH_2CH_2$-SH))
Cyclo-(Arg-Gly-Asp-D-Tyr-Orn($N^{\delta}$-CO-$CH_2CH_2$CO-NH-$CH_2CH_2$-SH))

Cyclo-(Arg-Gly-Asp-D-Phe-Orn($N^{\delta}$-CO-$CH_2CH_2$CO-NH-$CH_2CH_2$-SH))
Cyclo-(Arg-Gly-Asp-D-Trp-D-Orn($N^{\delta}$-CO-$CH_2CH_2$CO-NH-$CH_2CH_2$-SH))
Cyclo-(Arg-Gly-Asp-D-Tyr-D-Orn($N^{\delta}$-CO-$CH_2CH_2$CO-NH-$CH_2CH_2$-SH))
Cyclo-(Arg-Gly-Asp-D-Phe-D-Orn($N^{\delta}$-CO-$CH_2CH_2$CO-NH-$CH_2CH_2$-SH))
Cyclo-(Arg-Gly-Asp-D-Trp-Dab($N^{\gamma}$-CO-$CH_2CH_2$CO-NH-$CH_2CH_2$-SH))
Cyclo-(Arg-Gly-Asp-D-Tyr-Dab($N^{\gamma}$-CO-$CH_2CH_2$CO-NH-$CH_2CH_2$-SH))
Cyclo-(Arg-Gly-Asp-D-Trp-Dap($N^{\beta}$-CO-$CH_2CH_2$CO-NH-$CH_2CH_2$-SH))
Cyclo-(Arg-Gly-Asp-D-Tyr-Dap($N^{\beta}$-CO-$CH_2CH_2$CO-NH-$CH_2CH_2$-SH))
Cyclo-(Arg-Gly-Asp-D-Phe-Dap($N^{\beta}$-CO-$CH_2CH_2$CO-NH-$CH_2CH_2$-SH))
Cyclo-(Arg-Gly-Asp-D-Trp-D-Dap($N^{\beta}$-CO-$CH_2CH_2$CO-NH-$CH_2CH_2$-SH))
Cyclo-(Arg-Gly-Asp-D-Tyr-D-Dap($N^{\beta}$-CO-$CH_2CH_2$CO-NH-$CH_2CH_2$-SH))
Cyclo-(Arg-Gly-Asp-D-Phe-D-Dap($N^{\beta}$-CO-$CH_2CH_2$CO-NH-$CH_2CH_2$-SH)).

Analog Beispiel 2 erhält man durch Umsetzung der "G"-Cyclopeptide mit
a) "E" und b) Abspaltung der Schutzgruppen
die nachfolgenden Verbindungen

Cyclo-(Arg-Gly-Asp-D-Phe-Val-Lys($N^{\varepsilon}$-CO-$(CH_2)_5$-NH-CO-CH=$CH_2$))
Cyclo-(Arg-Gly-Asp-D-Trp-Lys($N^{\varepsilon}$-CO-$(CH_2)_5$-NH-CO-CH=$CH_2$))
Cyclo-(Arg-Gly-Asp-D-Tyr-Lys($N^{\varepsilon}$-CO-$(CH_2)_5$-NH-CO-CH=$CH_2$))
Cyclo-(Arg-Gly-Asp-D-Phe-D-Lys($N^{\varepsilon}$-CO-$(CH_2)_5$-NH-CO-CH=$CH_2$))
Cyclo-(Arg-Gly-Asp-D-Phe-Cys(S-CO-$(CH_2)_5$-NH-CO-CH=$CH_2$))
Cyclo-(Arg-Gly-Asp-D-Phe-Dab($N^{\gamma}$-CO-$(CH_2)_5$-NH-CO-CH=$CH_2$))
Cyclo-(Arg-Gly-Asp-D-Trp-D-Cys(S-CO-$(CH_2)_5$-NH-CO-CH=$CH_2$))
Cyclo-(Arg-Gly-Asp-D-Tyr-D-Cys(S-CO-$(CH_2)_5$-NH-CO-CH=$CH_2$))
Cyclo-(Arg-Gly-Asp-Phe-D-Lys($N^{\varepsilon}$-CO-$(CH_2)_5$-NH-CO-CH=$CH_2$))
Cyclo-(Arg-Gly-Asp-Trp-D-Lys($N^{\varepsilon}$-CO-$(CH_2)_5$-NH-CO-CH=$CH_2$))
Cyclo-(Arg-Gly-Asp-Tyr-D-Lys($N^{\varepsilon}$-CO-$(CH_2)_5$-NH-CO-CH=$CH_2$))
Cyclo-(Arg-Gly-Asp-Phe-D-Cys(S-CO-$CH_2CH_2$CO-NH-$CH_2CH_2$-SH))
Cyclo-(Arg-Gly-Asp-Phe-Dab($N^{\gamma}$-CO-$(CH_2)_5$-NH-CO-CH=$CH_2$))
Cyclo-(Arg-Gly-Asp-Trp-D-Cys(S-CO-$(CH_2)_5$-NH-CO-CH=$CH_2$))
Cyclo-(Arg-Gly-Asp-Tyr-D-Cys(S-CO-$(CH_2)_5$-NH-CO-CH=$CH_2$))
Cyclo-(Arg-Gly-Asp-D-Trp-Orn($N^{\delta}$-CO-$(CH_2)_5$-NH-CO-CH=$CH_2$))
Cyclo-(Arg-Gly-Asp-D-Tyr-Orn($N^{\delta}$-CO-$(CH_2)_5$-NH-CO-CH=$CH_2$))
Cyclo-(Arg-Gly-Asp-D-Phe-Orn($N^{\delta}$-CO-$(CH_2)_5$-NH-CO-CH=$CH_2$))
Cyclo-(Arg-Gly-Asp-D-Trp-D-Orn($N^{\delta}$-CO-$(CH_2)_5$-NH-CO-CH=$CH_2$))
Cyclo-(Arg-Gly-Asp-D-Tyr-D-Orn($N^{\delta}$-CO-$(CH_2)_5$-NH-CO-CH=$CH_2$))
Cyclo-(Arg-Gly-Asp-D-Phe-D-Orn($N^{\delta}$-CO-$(CH_2)_5$-NH-CO-CH=$CH_2$))
Cyclo-(Arg-Gly-Asp-D-Trp-Dab($N^{\gamma}$-CO-$(CH_2)_5$-NH-CO-CH=$CH_2$))
Cyclo-(Arg-Gly-Asp-D-Tyr-Dab($N^{\gamma}$-CO-$(CH_2)_5$-NH-CO-CH=$CH_2$))
Cyclo-(Arg-Gly-Asp-D-Trp-Dap($N^{\beta}$-CO-$(CH_2)_5$-NH-CO-CH=$CH_2$))
Cyclo-(Arg-Gly-Asp-D-Tyr-Dap($N^{\beta}$-CO-$(CH_2)_5$-NH-CO-CH=$CH_2$))
Cyclo-(Arg-Gly-Asp-D-Phe-Dap($N^{\beta}$-CO-$(CH_2)_5$-NH-CO-CH=$CH_2$))
Cyclo-(Arg-Gly-Asp-D-Trp-D-Dap($N^{\beta}$-CO-$(CH_2)_5$-NH-CO-CH=$CH_2$))
Cyclo-(Arg-Gly-Asp-D-Tyr-D-Dap($N^{\beta}$-CO-$(CH_2)_5$-NH-CO-CH=$CH_2$))
Cyclo-(Arg-Gly-Asp-D-Phe-D-Dap($N^{\beta}$-CO-$(CH_2)_5$-NH-CO-CH=$CH_2$)).

Beispiel für Zelladhäsionstest

Es wurde die Adhäsion von Maus-MC3T3 H1-Osteoblastenkulturen in vitro an RGD-Peptid-beschichteten Materialoberflächen untersucht. Dabei wurden 50.000 Zellen/$cm^2$ angesät und nach einstündiger Inkubation in serumfreiem Medium bei 37°/95 % Luftfeuchte der Anteil adherierter Zellen bestimmt.

$$\text{Zellanhaftungsrate [\%]} = \text{adherierte Zellen / angesäte Zellen} \times 100$$

Peptid: Zellanhaftungsrate [%]

Cyclo-(Arg-Gly-Asp-D-Phe-Lys($N^{\varepsilon}$-CO-$(CH_2)_5$-NH-CO-CH=$CH_2$)): 11,3;
Cyclo-(Arg-Gly-Asp-D-Phe-Lys($N^{\varepsilon}$- CO-$(CH_2)_5$-NH-CO-$(CH_2)_5$-NH-CO-CH=$CH_2$)): 92,4;
Cyclo-(Arg-Gly-Asp-D-Phe-Lys($N^{\varepsilon}$-CO-$CH_2$-O-$CH_2CH_2$-O-$CH_2CH_2$-NH-CO-$(CH_2)_5$-NH-CO-CH=$CH_2$)): 109,0;
Cyclo-(Arg-Gly-Asp-D-Phe-Lys($N^{\varepsilon}$-CO-$CH_2CH_2$CO-NH-$CH_2CH_2$-SH)): 86,2;
Cyclo-(Arg-Gly-Asp-D-Phe-Lys($N^{\varepsilon}$-(CO-$CH_2$-O-$CH_2CH_2$-O-$CH_2CH_2$-NH$)_2$-CO-$(CH_2)_5$-NH-CO-CH=$CH_2$)): 110,5.

**Patentansprüche**

**1.** Verbindungen der Formel I

R-Q-X I

worin

R cyclo-(Arg-Gly-Asp-Z), wobei Z in der Seitenkette an Q, oder falls Q fehlt, an X gebunden ist,

Q fehlt, -[CO-$R^1$-NH-$]_m$, -(NH-$R^1$-CO-$]_m$, -[CO-$R^1$-CO-$]_m$, -(CO-$CH_2$-O-$CH_2CH_2$-O-$CH_2CH_2$-NH-$)_n$, -(NH-$CH_2$-O-$CH_2CH_2$-O-$CH_2CH_2$-CO-$)_n$, -(NH-$CH_2CH_2$-O-$CH_2CH_2$-O-$CH_2$-CO-$)_n$ oder -(CO-$CH_2$-O-$CH_2CH_2$-O-$CH_2CH_2$-NH-$)_n$-[CO-$R^1$-NH-$]_m$,

X -CO-CH=$CH_2$, -CO-C($CH_3$)=$CH_2$, -NH-CH=$CH_2$, -NH-C($CH_3$)=$CH_2$ oder -NH-$(CH_2)_p$-$SR^{10}$,

Z jeweils unabhängig voneinander einen Aminosäurerest oder einen Di- oder Tripeptidrest, wobei die Aminosäuren unabhängig voneinander ausgewählt sind aus einer Gruppe bestehend aus Ala, Asn, Asp, Arg, Cys, Gln, Glu, Gly, His, Homo-Phe, Ile, Leu, Lys, Met, Phe, Phg, Pro, Ser, Thr, Trp, Tyr, Val oder M, wobei die genannten Aminosäuren auch derivatisiert sein können, und die Aminosäurereste über die α-Amino- und α-Carboxygruppen peptidartig miteinander verknüpft sind, und
wobei M immer enthalten ist,

M NH($R^8$)-CH($R^3$)-COOH,

$R^1$ fehlt oder $R^2$, $R^9$ , $R^2$-$R^9$-$R^2$, unsubstituiertes oder ein- oder zweifach durch $R^5$ substituiertes Phenylen, wobei die Kettenlänge von $R^5$ jeweils unabhängig voneinander ist,

$R^2$ Alkylen mit 1-10 C-Atomen, wobei 1 oder 2 Methylengruppen durch S, -CH=CH- oder -C=C- ersetzt sein können,

$R^3$ -$R^5$-$R^4$, -$R^6$-$R^4$, -$R^7$-$R^4$,

$R^4$ OH, $NH_2$, SH oder COOH,

$R^5$ Alkylen mit 1-6 C-Atomen,

$R^6$ Alkylenphenylen mit 7-14 C-Atomen,

$R^7$ Alkylenphenylalkylen mit 8-15 C-Atomen,

$R^8$ H, A oder Alkylenphenyl mit 7-12 C-Atomen,

$R^9$ Cycloalkylen mit 3-7 C-Atomen,

$R^{10}$ H oder eine S-Schutzgruppe,

A Alkyl mit 1-6 C-Atomen,

Hal F, Cl, Br oder I,

m,n jeweils unabhängig voneinander 0, 1, 2 oder 3

und

p 1, 2 oder 3 bedeuten,

wobei, sofern es sich um Reste optisch aktiver Aminosäuren und Aminosäurederivate handelt, sowohl die D- als auch die L-Formen eingeschlossen sind,
sowie deren Salze.

**2.** Verbindungen der Formel I gemäß Anspruch 1

a) Cyclo-(Arg-Gly-Asp-D-Phe-Lys($N^{\varepsilon}$-CO-$CH_2CH_2$CO-NH-$CH_2CH_2$-SH));
b) Cyclo-(Arg-Gly-Asp-D-Phe-Lys($N^{\varepsilon}$-CO-$CH_2CH_2$CO-NH-$CH_2CH_2$-SH)-Gly);
c) Cyclo-(Arg-Gly-Asp-D-Phe-Lys($N^{\varepsilon}$-CO-$(CH_2)_5$-NH-CO-CH=$CH_2$));
d) Cyclo-(Arg-Gly-Asp-D-Phe-Lys($N^{\varepsilon}$-CO-$(CH_2)_5$-NH-CO-$(CH_2)_5$-NH-CO-CH=$CH_2$));
e) Cyclo-(Arg-Gly-Asp-D-Phe-Lys($N^{\varepsilon}$-CO-$CH_2$-O-$CH_2CH_2$-O-$CH_2CH_2$-NH-CO-$(CH_2)_5$-NH-CO-CH=$CH_2$));
f) Cyclo-(Arg-Gly-Asp-D-Phe-Lys($N^{\varepsilon}$-(CO-$CH_2$-O-$CH_2CH_2$-O-$CH_2CH_2$-NH)$_2$-CO-$(CH_2)_5$-NH-CO-CH=$CH_2$));

sowie deren physiologisch unbedenklichen Salze.

**3.** Verfahren zur Herstellung von Verbindungen der Formel I nach Anspruch 1 sowie ihrer Salze, **dadurch gekennzeichnet, daß** man

(a) zur Herstellung von Verbindungen der Formel I,
worin

Q fehlt, -[CO-$R^1$-NH-]$_m$, -(CO-$CH_2$-O-$CH_2CH_2$-O-$CH_2CH_2$-NH-)$_n$,
oder
-(CO-$CH_2$-O-$CH_2CH_2$-O-$CH_2CH_2$-NH-)$_n$-[CO-$R^1$-NH-]$_m$,
X -CO-CH=$CH_2$ oder -CO-C($CH_3$)=$CH_2$

bedeuten,
und R die in Anspruch 1 angegebene Bedeutung hat,

i)
eine Verbindung der Formel II

R-H II

worin

R die in Anspruch 1 angegebene Bedeutung hat,

mit einer Verbindung der Formel III

L-Q-X III

worin

Q fehlt, -[CO-$R^1$-NH-]$_m$, -(CO-$CH_2$-O-$CH_2CH_2$-O-$CH_2CH_2$-NH-)$_n$,
oder
-(CO-$CH_2$-O-$CH_2CH_2$-O-$CH_2CH_2$-NH-)$_n$-(CO-$R^1$-NH-]$_m$,
X -CO-CH=$CH_2$ oder -CO-C($CH_3$)=$CH_2$

bedeuten,

und

L Cl, Br, I oder eine freie oder reaktionsfähig funktionell abgewandelte OH-Gruppe bedeutet,

umsetzt,
oder

ii)
eine Verbindung der Formel IV

$$R\text{-}Q\text{-}H \qquad IV$$

worin

Q fehlt, $-[CO-R^1-NH-]_m$ oder $-(CO-CH_2-O-CH_2CH_2-O-CH_2CH_2-NH-)_n$ oder $-(CO-CH_2-O-CH_2CH_2-O-CH_2CH_2-NH-)_n-(CO-R^1-NH-]_m$,

bedeuten und
R die in Anspruch 1 angegebene Bedeutung hat,
mit einer Verbindung der Formel V

$$L\text{-}X \qquad V$$

worin

X $-CO-CH=CH_2$ oder $-CO-C(CH_3)=CH_2$

bedeutet,
und

L Cl, Br, I oder eine freie oder reaktionsfähig funktionell abgewandelte OH-Gruppe bedeutet,

umsetzt,
oder

(b) zur Herstellung von Verbindungen der Formel I,
worin

Q fehlt, $-[NH-R^1-CO-]_m$, $-[CO-R^1-CO-]_m$, $-(NH-CH_2CH_2-O-CH_2CH_2-O-CH_2-CO-)_n$, oder $-(NH-CH_2-O-CH_2CH_2-O-CH_2CH_2-CO-)_n$,
X $-NH-CH=CH_2$, $-NH-C(CH_3)=CH_2$ oder $-NH-(CH_2)_p-SR^{10}$,
$R^{10}$ eine S-Schutzgruppe

bedeuten,
und R die in Anspruch 1 angegebene Bedeutung hat,

i)
eine Verbindung der Formel VI

$$R\text{-}L \qquad VI$$

worin

L Cl, Br, I oder eine freie oder reaktionsfähig funktionell abgewandelte OH-Gruppe bedeutet,

und R die in Anspruch 1 angegebene Bedeutung hat,
mit einer Verbindung der Formel VII

H-Q-X VII

worin

Q fehlt, $-[NH-R^1-CO-]_m$, $-(NH-CH_2-O-CH_2CH_2-O-CH_2CH_2-CO-)_n$ oder $-(NH-CH_2CH_2-O-CH_2CH_2-O-CH_2-CO-)_n$,
X $-NH-CH=CH_2$, $-NH-C(CH_3)=CH_2$ oder $-NH-(CH_2)_p-SR^{10}$,
$R^{10}$ eine S-Schutzgruppe

bedeuten,
umsetzt,
oder

ii)
eine Verbindung der Formel VIII

R-Q-L VIII

worin

Q fehlt, $-[NH-R^1-CO-]_m$, $-[CO-R^1-CO-]_m$, $-(NH-CH_2-O-CH_2CH_2-O-CH_2CH_2-CO-)_n$ oder $-(NH-CH_2CH_2-O-CH_2CH_2-O-CH_2-CO-)_n$,

L Cl, Br, I oder eine freie oder reaktionsfähig funktionell abgewandelte OH-Gruppe bedeutet,

und R die in Anspruch 1 angegebene Bedeutung hat,
mit einer Verbindung der Formel IX

H-X IX

worin

X $-NH-CH=CH_2$, $-NH-C(CH_3)=CH_2$ oder $-NH-(CH_2)_p-SR^{10}$,
$R^{10}$ eine S-Schutzgruppe

bedeuten,
umsetzt,
oder

c) daß man sie aus einem ihrer funktionellen Derivate durch Behandeln mit einem solvolysierenden oder hydrogenolysierenden Mittel in Freiheit setzt,

und/oder daß man eine basische oder saure Verbindung der Formel I durch Behandeln mit einer Säure oder Base in eines ihrer Salze überführt.

**4.** Verbindungen der Formel I nach Anspruch 1 zur kovalenten Bindung über die funktionalle Gruppe des Restes X an biokompatible Oberflächen.

**5.** Verbindungen nach Anspruch 4 als Integrininhibitoren zur selektiven Zellanreicherung an Implantaten.

**6.** Verbindungen nach Anspruch 4 oder 5 als Integrininhibitoren zur Behandlung von durch Implantate verursachten Erkrankungen, Defekten, Entzündungen und von osteolytischen Erkrankungen wie Osteoporose, Thrombose,

Herzinfarkt und Arteriosklerose, sowie zur Beschleunigung und Verstärkung des Integrationsprozesses des Implantats bzw. der biokompatiblen Oberfläche in das Gewebe.

**7.** Verwendung von Verbindungen nach Anspruch 6 zur Herstellung eines Arzneimittels zur Behandlung von durch Implantate verursachten Erkrankungen, Defekten, Entzündungen und von osteolytischen Erkrankungen wie Osteoporose, Thrombose, Herzinfarkt und Arteriosklerose, sowie zur Beschleunigung und Verstärkung des Integrationsprozesses des Implantats bzw. der biokompatiblen Oberfläche in das Gewebe.

**8.** Verwendung von Verbindungen der Formel I nach Anspruch 1, zur kovalenten Bindung an biokompatible Oberflächen.

**9.** Verwendung von Verbindungen der Formel I nach Anspruch 1 in der Affinitätschromatographie.

## Claims

**1.** Compounds of the formula I

R-Q-X    I

in which

R is cyclo-(Arg-Gly-Asp-Z), where Z is bonded in the side chain to Q, or if Q is absent, to X,

Q is absent, or is $-[CO-R^1-NH-]_m$, $-[NH-R^1-CO-]_m$, $-[CO-R^1-CO-]_m$, $-(CO-CH_2-O-CH_2CH_2-O-CH_2CH_2-NH-)_n$, $-(NH-CH_2-O-CH_2CH_2-O-CH_2CH_2-CO-)_n$, $-(NH-CH_2CH_2-O-CH_2CH_2-O-CH_2-CO-)_n$ or $-(CO-CH_2-O-CH_2CH_2-O-CH_2CH_2-NH-)_n-$ $[CO-R^1-NH-]_m$,

X is $-CO-CH=CH_2$, $-CO-C(CH_3)=CH_2$, $-NH-CH=CH_2$, $-NH-C(CH_3)=CH_2$ or $-NH-(CH_2)_p-SR^{10}$,

Z is in each case independently of one another an amino acid residue or a di- or tripeptide residue, where the amino acids independently of one another are selected from a group consisting of Ala, Asn, Asp, Arg, Cys, Gln, Glu, Gly, His, Homo-Phe, Ile, Leu, Lys, Met, Phe, Phg, Pro, Ser, Thr, Trp, Tyr, Val or M,
where the amino acids mentioned can also be derivatized, and the amino acid residues are linked to one another in peptide fashion via the α-amino- and α-carboxyl groups, and
where M is always present,

M is $NH(R^8)-CH(R^3)-COOH$,

$R^1$ is absent or is $R^2$, $R^9$, $R^2-R^9-R^2$, or phenylene which is unsubstituted or mono- or disubstituted by $R^5$, where the chain length of $R^5$ is in each case independent of one another,

$R^2$ is alkylene having 1-10 C atoms, where 1 or 2 methylene groups can be replaced by S, -CH=CH- or -C≡C-,

$R^3$ is $-R^5-R^4$, $-R^6-R^4$ or $-R^7-R^4$,

$R^4$ is OH, $NH_2$, SH or COOH,

$R^5$ is alkylene having 1-6 C atoms,

$R^6$ is alkylenephenylene having 7-14 C atoms,

$R^7$ is alkylenephenylalkylene having 8-15 C atoms,

$R^8$ is H, A or alkylenephenyl having 7-12 C atoms,

$R^9$ is cycloalkylene having 3-7 C atoms,

$R^{10}$ is H or an S protective group,

A is alkyl having 1-6 C atoms,

Hal is F, Cl, Br or I

m and n in each case independently of one another are 0, 1, 2 or 3

and

p is 1, 2 or 3,

where, if radicals of optically active amino acids and amino acid derivatives are concerned, both the D and the L forms are included,
and their salts.

**2.** Compounds of the formula I according to Claim 1

a) cyclo- (Arg-Gly-Asp-D-Phe-Lys($N^{\varepsilon}$-CO-$CH_2CH_2$CO-NH-$CH_2CH_2$-SH));
b) cyclo- (Arg-Gly-Asp-D-Phe-Lys($N^{\varepsilon}$-CO-$CH_2CH_2$CO-NH-$CH_2CH_2$-SH)-Gly);
c) cyclo-(Arg-Gly-Asp-D-Phe-Lys ($N^{\varepsilon}$-CO- $(CH_2)_5$-NH-CO-CH=$CH_2$));
d) cyclo-(Arg-Gly-Asp-D-Phe-Lys($N^{\varepsilon}$-CO-$(CH_2)_5$-NH-CO-$(CH_2)_5$-NH-CO-CH=$CH_2$));
e) cyclo-(Arg-Gly-Asp-D-Phe-Lys($N^{\varepsilon}$-CO-$CH_2$-O-$CH_2CH_2$-O-$CH_2CH_2$-NH-CO-$(CH_2)_5$-NH-CO-CH=$CH_2$));
f) cyclo-(Arg-Gly-Asp-D-Phe-Lys($N^{\varepsilon}$-(CO-$CH_2$-O-$CH_2CH_2$-O-$CH_2CH_2$-NH$)_2$-CO-$(CH_2)_5$-NH-CO-CH=$CH_2$));

and their physiologically acceptable salts.

**3.** Process for the preparation of compounds of the formula I according to Claim 1 and of their salts, **characterized in that**

(a) for the preparation of compounds of the formula I
in which

Q is absent, or is - [CO-$R^1$-NH-$]_m$, -(CO-$CH_2$-O-$CH_2CH_2$-O-$CH_2CH_2$-NH-$)_n$
or
-(CO-$CH_2$-O-$CH_2CH_2$-O-$CH_2CH_2$-NH-$)_n$-[CO-$R^1$-NH-$]_m$,
X is -CO-CH=$CH_2$ or -CO-C($CH_3$)=$CH_2$,

and R has the meaning indicated in Claim 1,

i)
a compound of the formula II

R-H II

in which

R has the meaning indicated in Claim 1,

is reacted with a compound of the formula III

L-Q-X III

in which

Q is absent, or is - [CO-$R^1$-NH-$]_m$, -(CO-$CH_2$-O-$CH_2CH_2$-O-$CH_2CH_2$-NH-$)_n$

or
$-(CO-CH_2-O-CH_2CH_2-O-CH_2CH_2-NH-)_n- [CO-R^1-NH-]_m$,
X is $-CO-CH=CH_2$ or $-CO-C(CH_3)=CH_2$,

and

L is Cl, Br, I or a free or reactive functionally modified OH group,

or

ii)
a compound of the formula IV

$$R-Q-H \quad IV$$

in which

Q is absent, or is $-[CO-R^1-NH-]_m$, $-(CO-CH_2-O-CH_2CH_2-O-CH_2CH_2-NH-)_n$ or $-(CO-CH_2-O-CH_2CH_2-O-CH_2CH_2-NH-)_n- [CO-R^1-NH-]_m$,

and
R has the meaning indicated in Claim 1,
is reacted with a compound of the formula V

$$L-X \quad V$$

in which

X is $-CO-CH=CH_2$ or $-CO-C(CH_3)=CH_2$,

and

L is Cl, Br, I or a free or reactive functionally modified OH group,

or

(b) for the preparation of compounds of the formula I
in which

Q is absent, or is $- [NH-R^1-CO-]_m$, $- [CO-R^1-CO-]_m$,
$-(NH-CH_2CH_2-O-CH_2CH_2-O-CH_2-CO-)_n$ or
$-(NH-CH_2-O-CH_2CH_2-O-CH_2CH_2-CO-)_n$,
X is $-NH-CH=CH_2$, $-NH-C (CH_3) =CH_2$ or $-NH-(CH_2)_p-SR^{10}$,
$R^{10}$ is an S protective group,

and R has the meaning indicated in Claim 1,

i)
a compound of the formula VI

$$R-L \quad VI$$

in which

L is Cl, Br, I or a free or reactive functionally modified OH group,

and R has the meaning indicated in Claim 1,
is reacted with a compound of the formula VII

$$H\text{-}Q\text{-}X \qquad VII$$

in which

Q is absent, or is - $[NH\text{-}R^1\text{-}CO\text{-}]_m$, $-(NH\text{-}CH_2\text{-}O\text{-}CH_2CH_2\text{-}O\text{-}CH_2CH_2\text{-}CO\text{-})_n$ or $-(NH\text{-}CH_2CH_2\text{-}O\text{-}CH_2CH_2\text{-}O\text{-}CH_2\text{-}CO\text{-})_n$,
X is $-NH\text{-}CH{=}CH_2$, $-NH\text{-}C(CH_3){=}CH_2$ or $-NH\text{-}(CH_2)_p\text{-}SR^{10}$,
$R^{10}$ is an S protective group,

or

ii)
a compound of the formula VIII

$$R\text{-}Q\text{-}L \qquad VIII$$

in which

Q is absent, or is $-[NH\text{-}R^1\text{-}CO\text{-}]_m$, $-[CO\text{-}R^1\text{-}CO\text{-}]_m$, $-(NH\text{-}CH_2\text{-}O\text{-}CH_2CH_2\text{-}O\text{-}CH_2CH_2\text{-}CO\text{-})_n$ or $-(NH\text{-}CH_2CH_2\text{-}O\text{-}CH_2CH_2\text{-}O\text{-}CH_2\text{-}CO\text{-})_n$,
L is Cl, Br, I or a free or reactive functionally modified OH group,

and R has the meaning indicated in Claim 1,
is reacted with a compound of the formula IX

$$H\text{-}X \qquad IX$$

in which

X is $-NH\text{-}CH{=}CH_2$, $-NH\text{-}C(CH_3){=}CH_2$ or $-NH\text{-}(CH_2)_p\text{-}SR^{10}$,
$R^{10}$ is an S protective group,

or

(c) **in that** it is liberated from one of its functional derivatives by treating with a solvolysing or hydrogenolysing agent,

and/or **in that** a basic or acidic compound of the formula I is converted into one of its salts by treating with an acid or base.

**4.** Compounds of the formula I according to Claim 1 for covalent bonding via the functional group of the radical X to biocompatible surfaces.

**5.** Compounds according to Claim 4 as integrin inhibitors for selective cell enrichment on implants.

**6.** Compounds according to Claim 4 or 5 as integrin inhibitors for the treatment of disorders caused by implants, defects, inflammations and of osteolytic disorders such as osteoporosis, thrombosis, cardiac infarct and arteriosclerosis, and also for the acceleration and reinforcement of the integration process of the implant or of the biocompatible surface into the tissue.

**7.** Use of compounds according to Claim 6 for the production of a medicament for the treatment of disorders caused by implants, defects, inflammations and of osteolytic disorders such as osteoporosis, thrombosis, cardiac infarct

and arteriosclerosis, and also for the acceleration and reinforcement of the integration process of the implant or of the biocompatible surface into the tissue.

**8.** Use of compounds of the formula I according to Claim 1, for covalent bonding to biocompatible surfaces.

**9.** Use of compounds of the formula I according to Claim 1 in affinity chromatography.

**Revendications**

**1.** Composés de formule I

$$R\text{-}Q\text{-}X \qquad I$$

dans laquelle

R représente un groupe cyclo-(Arg-Gly-Asp-Z), Z étant lié à Q dans la chaîne latérale, ou si Q est absent, à X,
Q est absent, représente un groupe $-[CO-R^1-NH-]_m$, $-[NH-R^1-CO-]_m$, $-[CO-R^1-CO-]_m$, $-(CO-CH_2-O-CH_2CH_2-O-CH_2CH_2-NH-)_n$, $-(NH-CH_2-O-CH_2CH_2-O-CH_2CH_2-CO-)_n$, $-(NH-CH_2CH_2-O-CH_2CH_2-O-CH_2-CO-)_n$ ou $-(CO-CH_2-O-CH_2CH_2-O-CH_2CH_2-NH-)_n-[CO-R^1-NH-]_m$,
X représente un groupe $-CO-CH=CH_2$, $-CO-C(CH_3)-CH_2$, $-NH-CH=CH_2$, $-NH-C(CH_3)=CH_2$ ou $-NH-(CH_2)_pSR^{10}$,
Z représente, chacun indépendamment l'un de l'autre, un radical d'acide aminé ou un radical de di ou tripeptide, les acides aminés, indépendamment l'un de l'autre, étant choisis dans un groupe constitué par Ala, Asn, Asp, Arg, Cys, Gln, Glu, Gly, His, homo-Phe, Ile, Leu, Lys, Met, Phe, Phg, Pro, Ser, Thr, Trp, Tyr, Val ou M,

les acides aminés cités pouvant également être dérivatisés, et les radicaux d'acides aminés étant reliés ensemble de façon peptidique par l'intermédiaire des groupes α-amino et α-carboxy et

M est toujours présent,
M représente un groupe $NH(R^8)-CH(R^3)-COOH$,
$R^1$ est absent ou représente $R^2$, $R^9$, $R^2-R^9-R^2$, un groupe phénylène non substitué ou substitué une ou deux fois par $R^5$, la longueur de chaîne de $R^5$ étant indépendante l'une de l'autre,
$R^2$ représente un groupe alkylène portant 1 à 10 atomes de C, 1 ou 2 groupes méthylène pouvant être remplacés par un groupe S, -CH=CH- ou —C≡C—,
$R^3$ représente $-R^5-R^4$, $-R^6-R^4$, $-R^7-R^4$,
$R^4$ représente un groupe OH, $NH_2$, SH ou COOH,
$R^5$ représente un groupe alkylène ayant 1 à 6 atomes de C,
$R^6$ représente un groupe alkylène-phénylène ayant 7 à 14 atomes de C,
$R^7$ représente un groupe alkylène-phénylalkylène ayant 8 à 15 atomes de C,
$R^8$ représente un atome de H, A ou un groupe alkylène phényle ayant 7 à 12 atomes de C,
$R^9$ représente un groupe cycloalkylène ayant 3 à 7 atomes de C,
$R^{10}$ représente un atome de H ou un groupe protecteur de S,
A représente un groupe alkyle ayant 1 à 6 atomes de C,
Hal représente un atome de F, Cl, Br ou I,
m, n valent chacun, indépendamment l'un de l'autre 0, 1, 2 ou 3

et

P vaut 1, 2 ou 3,

aussi bien les formes D et les formes L étant incluses s'il s'agit de radicaux d'acides aminés et de dérivés d'acides aminés optiquement actifs,
ainsi que leurs sels.

**2.** Composés de formule I selon la revendication 1,

a) Cyclo-(Arg-Gly-Asp-D-Phe-Lys ($N^{\varepsilon}$-CO-$CH_2CH_2$CO-NH-$CH_2CH_2$SH)) ;
b) Cyclo-(Arg-Gly-Asp-D-Phe-Lys ($N^{\varepsilon}$-CO-$CH_2CH_2$CO-NH-$CH_2CH_2$SH)-Gly) ;
c) Cyclo-(Arg-Gly-Asp-D-Phe-Lys ($N^{\varepsilon}$-CO-$(CH_2)_5$-NH-CO-CH=$CH_2$)) ;
d) Cyclo-(Arg-Gly-Asp-D-Phe-Lys ($N^{\varepsilon}$-CO-$(CH_2)_5$-NH-CO-$(CH_2)_5$NH-CO-CH=$CH_2$)) ;
e) Cyclo-(Arg-Gly-Asp-D-Phe-Lys ($N^{\varepsilon}$-CO-$CH_2$-O-$CH_2CH_2$-O-$CH_2CH_2$-NH-CO-$(CH_2)_5$-NH-CO-CH=$CH_2$)) ;
f) Cyclo-(Arg-Gly-Asp-D-Phe-Lys ($N^{\varepsilon}$-(CO-$CH_2$-O-$CH_2CH_2$-O$CH_2CH_2$-NH$)_2$-CO-$(CH_2)_5$-NH-CO-CH=$CH_2$)) ;

ainsi que leurs sels physiologiquement inoffensifs.

**3.** Procédé de fabrication de composés de formule I selon la revendication 1 ainsi que leurs sels, **caractérisé en ce que**

(a) pour fabriquer des composés de formule I,
dans laquelle

Q est absent, représente un groupe -[CO-$R^1$-NH-$]_m$,
-(CO-$CH_2$-O-$CH_2CH_2$-O-$CH_2CH_2$-NH-$)_n$,
ou
(CO-$CH_2$-O-$CH_2CH_2$-O-$CH_2CH_2$-NH- n- [CO-$R^1$-NH-$]_m$,
X représente un groupe -CO-CH=$CH_2$ ou -CO-C($CH_3$)=$CH_2$,
et R a la signification indiquée dans la revendication 1.

i)
on convertit
un composé de formule II

R-H II

dans laquelle

R a la signification indiquée dans la revendication 1,

avec un composé de formule III

L-Q-X III

dans laquelle

Q est absent, représente un groupe -[CO-$R^1$-NH-$]_m$,
-(CO-$CH_2$-O-$CH_2CH_2$-O-$CH_2CH_2$-NH-$)_n$,
ou
-(CO-$CH_2$-O-$CH_2CH_2$-O-$CH_2CH_2$-NH-$)_n$- [CO-$R^1$-NH-$]_m$,
X représente un groupe -CO-CH=$CH_2$ ou -CO-C($CH_3$)=$CH_2$,

et

L représente un atome de Cl, Br, I ou un groupe OH libre ou fonctionnellement modifié capable d'entrer en réaction,

ou

ii)
un composé de formule IV

R-Q-H IV

dans laquelle

Q est absent, représente un groupe $-[CO-R^1-NH-]_m$ ou $-(CO-CH_2-O-CH_2CH_2-O-CH_2CH_2-NH-)_n$ ou $-(CO-CH_2-O-CH_2CH_2-O-CH_2CH_2-NH-)_n- [CO-R^1-NH-]_m$,
et
R a la signification indiquée dans la revendication 1

avec un composé de formule V

L-X V

dans laquelle

X représente un groupe $-CO-CH=CH_2$ ou $-CO-C(CH_3) =CH_2$

et

L représente un atome de Cl, Br, I ou un groupe OH libre ou fonctionnellement modifié capable d'entrer en réaction,

ou
(b) pour fabriquer des composés de formule I,
dans laquelle

Q est absent, représente un groupe $-[NH-R^1-CO]_m$, $-[CO-R^1-CO-]_m$, $-(NH-CH_2CH_2-O-CH_2CH_2-O-CH_2CO-)_n$ ou $-(NH-CH_2-O-CH_2CH_2-O-CH_2CH_2-CO-)_n$,
X représente un groupe $-NH-CH=CH_2$, $-NH-C-CH_3)=CH_2$ ou $-NH-(CH_2)_p-SR^{10}$,
$R^{10}$ représente un groupe protecteur de S,
et R a la signification indiquée dans la revendication 1,

i)
on convertit un composé de formule VI

R-L VI

dans laquelle

L représente un atome de Cl, Br, I ou un groupe OH libre ou fonctionnellement modifié capable d'entrer en réaction,
et R a la signification indiquée dans la revendication 1,

avec un composé de formule VII

H-Q-X VII

dans laquelle

Q est absent, représente un groupe $-[NH-R^1-CO]_m$, $-(NH-CH_2-O-CH_2CH_2-O-CH_2CH_2-CO-)_n$ ou $-(NH-CH_2CH_2-O-CH_2CH_2-O-CH_2CO-)_n$,
X représente un groupe $-NH-CH=CH_2$, $-NH-C-(CH_3) =CH_2$ ou $-NH-(CH_2)_p-SR^{10}$,
$R^{10}$ représente un groupe protecteur de S,

ou
ii)
on convertit un composé de formule VIII

R-Q-L VIII

dans laquelle

Q est absent, représente un groupe $-[NH-R^1-CO]_m$, $-[CO-R^1-CO-]_m$, $-(NH-CH_2-O-CH_2CH_2-O-CH_2CH_2-CO-)_n$ ou $-(NH-CH_2CH_2-O-CH_2CH_2-O-CH_2-CO-)_n$,
L représente un atome de Cl, Br, 1 ou un groupe OH libre ou fonctionnellement modifié capable d'entrer en réaction,
et R a la signification indiquée dans la revendication 1,

avec un composé de formule IX

H-X IX

dans laquelle

X représente un groupe $-NH-CH=CH_2$, $-NH-C-CH_3)=CH_2$ ou $-NH-(CH_2)_p-SR^{10}$,
$R^{10}$ un groupe protecteur de S,

ou

c) **en ce qu'**on les libère de l'un de leurs dérivés fonctionnels par un traitement avec un agent solvolysant ou hydrogénolysant,

et/ou en qu'on transforme un composé basique ou acide de formule I en un de ses sels par un traitement avec un acide ou une base.

**4.** Composés de formule I selon la revendication 1 pour la liaison covalente à des surfaces biocompatibles par l'intermédiaire d'un groupe fonctionnel du radical X.

**5.** Composés selon la revendication 4 en tant qu'inhibiteurs de l'intégrine pour l'enrichissement cellulaire sélectif sur des implants.

**6.** Composés selon la revendication 4 ou 5 en tant qu'inhibiteurs de l'intégrine pour le traitement de maladies, troubles, inflammations et maladies ostéolytiques provoquées par des implants, telles que l'ostéoporose, la thrombose, l'infarctus du myocarde et l'artériosclérose ainsi que pour l'accélération et le renforcement du processus d'intégration de l'implant ou de la surface biocompatible dans le tissu.

**7.** Utilisation de composés selon la revendication 6 pour la fabrication d'un médicament pour le traitement de maladies, troubles, inflammations et maladies ostéolytiques provoquées par des implants, telles que l'ostéoporose, la thrombose, l'infarctus du myocarde et l'artériosclérose ainsi que pour l'accélération et le renforcement du processus d'intégration de l'implant ou de la surface biocompatible dans le tissu.

**8.** Utilisation de composés de formule I selon la revendication 1 pour la liaison covalente à des surfaces biocompatibles.

**9.** Utilisation de composés de formule I selon la revendication 1 dans la chromatographie d'affinité.